# EUROPEAN PATENT APPLICATION

(11) **EP 4 610 350 A1**
(43) Date of publication of application: **03.09.2025**
(21) Application number: 23882991.5
(22) Date of filing: 18.10.2023
(51) Int. Cl.: C12N 9/00, C12N 15/70, C12N 15/77, C12P 13/22, C12N 9/88, C12N 9/90, C07K 14/245, C12N 9/12, C12N 15/52

(54) **NOVEL PHENYLALANINE:H+ SYMPORTER PHEP VARIANT, AND METHOD FOR PRODUCING L-AROMATIC AMINO ACIDS USING SAME**

(30) Priority: 24.10.2022 KR 20220137248; 13.10.2023 KR 20230136996
(71) Applicant: Daesang Corporation, Seoul 02586 (KR)
(72) Inventor: KIM, Hyun Young, Seoul 07789 (KR); CHA, Joy, Seoul 07789 (KR); JO, Young Il, Seoul 07789 (KR)
(74) Representative: Ipsilon
(86) International application number: PCT/KR2023/016161
(87) International publication number: WO 2024/090884

(57) **Abstract**

The present invention relates to a novel variant of phenylalanine:H+ symporter PheP and a method of producing L-aromatic amino acids using the same. The phenylalanine:H+ symporter PheP variant is obtained by substituting one or more amino acids in the amino acid sequence constituting phenylalanine:H+ symporter PheP to change the activity of the protein, and a recombinant microorganism comprising the variant is capable of efficiently producing L-tryptophan, L-phenylalanine, or L-tyrosine.

## Description

### Technical Field

The present invention relates to a novel variant of phenylalanine:H+ symporter PheP and a method of producing an L-aromatic amino acid using the same.

### Background Art

Amino acids are classified into hydrophobic, hydrophilic, basic, and acidic amino acids, depending on the nature of their side chains, and among them, amino acids having a benzene ring are called aromatic amino acids. Aromatic amino acids include phenylalanine, tyrosine, and tryptophan. Phenylalanine and tryptophan are essential amino acids that are not synthesized *in vivo* and correspond to high-value-added industries that form a global market worth $300 billion annually.

For the production of aromatic amino acids, either naturally occurring wild-type strains or mutant strains modified to have an increased ability to produce amino acids may be used. In recent years, in order to improve the efficiency of production of aromatic amino acids, there has been development of a variety of recombinant strains or mutant strains having excellent L-aromatic amino acid productivity by applying genetic recombination technology to microorganisms such as *Escherichia coli* and *Corynebacterium,* which are widely used in the production of useful substances such as L-amino acids, and methods of producing L-aromatic amino acids using the same. In particular, there have been attempts to increase the production of L-aromatic amino acids by targeting genes such as enzymes, transcription factors and transport proteins, which are involved in the biosynthetic pathways of L-aromatic amino acids, or by inducing mutations in promoters that regulate the expression of these genes. However, there are dozens of types of proteins such as enzymes, transcription factors and transport proteins, which are involved directly or indirectly in the production of L-aromatic amino acids, and thus much research is still needed on the increase in L-aromatic amino acid productivity by changes in the activity of these proteins.

### [Prior Art Documents]

### [Patent Documents]

Korean Patent No. 10-1830002

### DISCLOSURE

### Technical Problem

An object of the present invention is to provide a novel variant of phenylalanine:H+ symporter PheP.

Another object of the present invention is to provide a polynucleotide encoding the variant.

Still another object of the present invention is to provide a transformant comprising the variant or the polynucleotide.

Yet another object of the present invention is to provide a method of producing an L-aromatic amino acid using the transformant.

### Technical Solution

One aspect of the present invention provides a phenylalanine:H+ symporter PheP variant consisting of the amino acid sequence of SEQ ID NO: 4 in which glutamic acid (Glu) at position 272 in the amino acid sequence of SEQ ID NO: 2 is substituted with lysine (Lys).

The "phenylalanine:H+ symporter PheP" as used in the present invention is also called phenylalanine-specific permease and functions to transport phenylalanine across the cytoplasmic membrane. The phenylalanine:H+ symporter PheP in the present invention may be a polypeptide encoded by the pheP gene and having phenylalanine:H+ symporter PheP activity, without being limited thereto.

Information on the nucleic acid and protein sequences of the phenylalanine:H+ symporter PheP is available from known sequence databases (e.g., GenBank, UniProt).

According to one embodiment of the present invention, the phenylalanine:H+ symporter PheP may be encoded by the nucleotide sequence of SEQ ID NO: 1 and may consist of the amino acid sequence of SEQ ID NO: 2.

The amino acid sequence of the phenylalanine:H+ symporter PheP according to the present invention or the nucleotide sequence encoding the same may comprise a nucleotide sequence or amino acid sequence having at least 70%, 75%, 80%, 85%, 90%, 95%, 96%, 97%, 98%, 99% or 100% homology or identity to the amino acid sequence of SEQ ID NO: 2 or the nucleotide sequence of SEQ ID NO: 1. As used herein, the term "homology" or "identity" means the percentage of identity between two sequences, which is determined by aligning the reference nucleotide sequence or amino acid sequence and any other nucleotide sequence or amino acid sequence to correspond to each other as much as possible and analyzing the aligned sequences.

According to one embodiment of the present invention, the phenylalanine:H+ symporter PheP or the gene encoding the same may be derived from wild-type *Escherichia coli.*

As used in the present invention, the term "variant" refers to a protein that has an amino acid sequence different from the original amino acid sequence of the protein due to mutation in the nucleotide sequence of the gene encoding the protein. More specifically, the mutation in the gene sequence is caused by the substitution, insertion, deletion or the like of one or more bases or nucleotides in the sequence of the gene, and a polypeptide or protein translated from the gene is a protein variant that has an amino acid sequence different from the amino acid sequence before mutation by the conservative substitution and/or modification of one or more amino acids at the N-terminus, C-terminus of and/or within the amino acid sequence, but retains the functions or properties of the protein before mutation. As used herein, the term "conservative substitution" means substituting one amino acid with another amino acid having similar structural and/or chemical properties. The conservative substitution may have little or no impact on the activity of the protein or polypeptide. The amino acid is selected from among alanine (Ala), isoleucine (Ile), valine (Val), leucine (Leu), methionine (Met), asparagine (Asn), cysteine (Cys), glutamine (Gln), serine (Ser), threonine (Thr), phenylalanine (Phe), tryptophan (Trp), tyrosine (Tyr), aspartic acid (Asp), glutamic acid (Glu), arginine (Arg), histidine (His), lysine (Lys), glycine (Gly), and proline (Pro) .

In addition, some variants include those in which one or more portions, such as an N-terminal leader sequence or transmembrane domain, have been removed, or those in which a portion has been removed from the N- and/or C-terminus of a mature protein.

The variant may have increased (enhanced), unchanged, or decreased (weakened) ability compared to that of the protein before mutation. Here, the term "increased or enhanced" includes: a case in which the activity of the protein itself has increased compared to the activity of the protein before mutation; a case in which the overall activity of the protein in the cell is higher than that in the wild-type strain or the strain expressing the protein before mutation due to increased expression or translation of the gene encoding the protein; and a combination thereof. In addition, the term "decreased or weakened" includes: a case in which the activity of the protein itself has decreased compared to the activity of the protein before mutation; a case in which the overall activity of the protein in the cell is lower than that in the wild-type strain or the strain expressing the protein before mutation due to reduced expression or translation of the gene encoding the protein; and a combination thereof. In the present invention, the term "variant" may be used interchangeably with terms such as variant type, modification, variant polypeptide, mutated protein, mutation, and the like.

The phenylalanine:H+ symporter PheP variant according to the present invention may comprise an amino acid sequence having at least 70%, 75%, 80%, 85%, 90%, 95%, 96%, 97%, 98%, 99% or 100% homology or identity to the amino acid sequence of SEQ ID NO: 4, and any amino acid sequence that retains the functions or properties of the variant may be included without limitation.

Another aspect of the present invention provides a polynucleotide encoding the phenylalanine:H+ symporter PheP variant.

As used in the present invention, the term "polynucleotide" refers to a DNA or RNA strand having a certain length or more, which is a long-chain polymer of nucleotides formed by linking nucleotide monomers via covalent bonds. More specifically, the term "polynucleotide" refers to a polynucleotide fragment encoding the protein variant.

According to one embodiment of the present invention, the polynucleotide may comprise a nucleotide sequence encoding the amino acid sequence of SEQ ID NO: 4. For example, it may comprise the nucleotide sequence of SEQ ID NO: 3.

Still another aspect of the present invention provides a vector comprising a polynucleotide encoding the phenylalanine:H+ symporter PheP variant.

Yet another aspect of the present invention provides a transformant comprising the phenylalanine:H+ symporter PheP variant or the polynucleotide.

As used in the present invention, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and regulatory sequences thereof are functionally linked together in a manner enabling gene expression, and the "regulatory elements" include a promoter for initiating transcription, any operator sequence for regulating transcription, a sequence encoding suitable mRNA ribosome-binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, λMBL3, λMBL4, λIXII, λASHII, λAPII, λt10, λt11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The recombinant vector used in the present invention may be constructed using a prokaryotic or eukaryotic cell as a host, and may replicate regardless of the genome of the host cell or may be integrated into the genome itself. The host cell may contain a replication origin, which is a particular nucleotide sequence which enables the vector to replicate in the host cell and from which replication starts. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, and T7 promoter), a ribosome binding site for initiation of translation, and a transcription/translation termination sequence. When a eukaryotic cell is used as a host, the vector comprises a replication origin operating in the eukaryotic cell, and examples of the replication origin include, but are not limited to, an f1 replication origin, an SV40 replication origin, a pMB1 replication origin, an adeno replication origin, an AAV replication origin, and a BBV replication origin. In addition, the recombinant vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV-tk promoter), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, ampicillin, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

Where the vector is transformed into prokaryotic cells to generate recombinant microorganisms, examples of host cells that may be used include, but are not limited to, *E. coli* sp. strains such as *E. coli* JM109, *E*. *coli* BL21, *E. coli* RR1, *E. coli* LE392, *E. coli* B, *E. coli* X 1776, *E*. *coli* W3110, and *E*. *coli* XL1-Blue, *Corynebacterium* sp. strains, *Bacillus* sp. strains such as *Bacillus subtilis* and *Bacillus thuringiensis,* and various Enterobacteriaceae strains such as *Salmonella typhimurium, Serratia marcescens,* and *Pseudomonas* species.

Where the vector is transformed into eukaryotic cells to generate recombinant microorganisms, examples of host cells that may be used include, but are not limited to, yeast (e.g., *Saccharomyces cerevisiae*), insect cells, and plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be inserted into the chromosome of the host cell, or may exist extrachromosomally, without being limited thereto.

The transformant may include a cell transfected, transformed, or infected with the recombinant vector of the present invention *in vivo* or *in vitro,* and may be used in the same sense as a recombinant host cell, a recombinant cell, or a recombinant microorganism.

According to one embodiment of the present invention, the transformant may be an *Escherichia* sp. strain or a *Corynebacterium* sp. strain.

The *Escherichia* sp. strain may be *Escherichia coli, Escherichia albertii, Escherichia blattae, Escherichia fergusonii, Escherichia hermannii,* or *Escherichia vulneris,* without being limited thereto.

The *Corynebacterium* sp. strain may be *Corynebacterium glutamicum, Corynebacterium crudilactis, Corynebacterium deserti, Corynebacterium callunae, Corynebacterium suranareeae, Corynebacterium lubricantis, Corynebacterium doosanense, Corynebacterium efficiens, Corynebacterium uterequi, Corynebacterium stationis, Corynebacterium pacaense, Corynebacterium singulare, Corynebacterium humireducens, Corynebacterium marinum, Corynebacterium halotolerans, Corynebacterium spheniscorum, Corynebacterium freiburgense, Corynebacterium striatum, Corynebacterium canis, Corynebacterium ammoniagenes, Corynebacterium renale, Corynebacterium pollutisoli, Corynebacterium imitans, Corynebacterium caspium, Corynebacterium testudinoris, Corynebacaterium pseudopelargi,* or *Corynebacterium flavescens,* without being limited thereto.

The transformant in the present invention may be a strain either comprising the above-described phenylalanine:H+ symporter PheP variant or a polynucleotide encoding the same or comprising the vector comprising the same, a strain expressing the phenylalanine:H+ symporter PheP variant or the polynucleotide, or a strain having activity for the phenylalanine:H+ symporter PheP variant, without being limited thereto.

The transformant of the present invention may comprise other protein variants or genetic mutations, in addition to the phenylalanine:H+ symporter PheP variant.

According to one embodiment of the present invention, the transformant may have the ability to produce an L-aromatic amino acid.

The transformant may naturally have the ability to produce an L-aromatic amino acid or may be one artificially endowed with the ability to produce an L-aromatic amino acid.

According to one embodiment of the present invention, the transformant may have an increased ability to produce an L-aromatic amino acid, due to a change in the activity of phenylalanine:H+ symporter PheP.

As used in the present invention, the term "increased ability to produce" means that L-aromatic amino acid productivity has increased compared to that of the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Escherichia* sp. or *Corynebacterium* sp. strain or an *Escherichia* sp. or *Corynebacterium* sp. strain mutated from the wild-type strain.

The transformant according to the present invention exhibits an increased ability to produce an L-aromatic amino acid compared to the strain (parent strain) containing the protein before mutation, due to the change in phenylalanine:H+ symporter PheP activity caused by introduction of the phenylalanine:H+ symporter PheP variant thereinto. More specifically, the amount of L-aromatic amino acid produced by the transformant may be at least 1%, 5%, 10%, 15%, 20%, 25%, 30%, 35%, 40%, 45%, 50%, 55%, 60%, 65%, 70%, 75%, 80%, 85%, 90%, 95%, or 100% higher than that produced by the parent strain, or may be 1.1-fold, 1.5-fold, 2-fold, 2.5-fold, 3-fold, 3.5-fold, 4-fold, 4.5-fold, 5-fold, 5.5-fold, 6-fold, 6.5-fold, 7-fold, 7.5-fold, 8-fold, 8.5-fold, 9-fold, 9.5-fold, or 10-fold higher than that produced by the parent strain, without being limited thereto. For example, the amount of L-aromatic amino acid produced by the transformant comprising the phenylalanine:H+ symporter PheP variant may be at least 5%, specifically 5 to 50% (preferably 7 to 40%) higher than that produced by the parent strain.

A composition comprising the transformant according to the present invention may be used as a composition for producing an L-aromatic amino acid.

Still yet another aspect of the present invention provides a method for producing an L-aromatic amino acid, comprising steps of: culturing the transformant in a medium; and recovering an L-aromatic amino acid from the transformant or the medium in which the transformants has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For culture media for *Escherichia* sp. strains or *Corynebacterium* sp. strains, reference may be made to, but not limited to, a known document (Manual of Methods for General Bacteriology, American Society for Bacteriology, Washington D.C., USA, 1981) .

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering an L-aromatic amino acid from the cultured transformant or the medium in which the transformant has been cultured, the produced L-aromatic amino acid may be collected or recovered from the medium using a suitable method known in the art depending on the culture method. Examples of a method that may be used to recover the produced L-aromatic amino acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering an L-aromatic amino acid may be performed by centrifuging the culture medium at a low speed to remove biomass and separating the obtained supernatant through ion-exchange chromatography.

According to one embodiment of the present invention, the step of recovering an L-aromatic amino acid may include a process of purifying the L-aromatic amino acid.

According to one embodiment of the present invention, the L-aromatic amino acid may be at least one selected from the group consisting of L-tryptophan, L-phenylalanine, and L-tyrosine.

### Advantageous Effects

The phenylalanine:H+ symporter PheP variant according to the present invention is obtained by substituting one or more amino acids in the amino acid sequence constituting phenylalanine:H+ symporter PheP to change the activity of the protein, and a recombinant microorganism comprising the variant is capable of efficiently producing L-tryptophan, L-phenylalanine, or L-tyrosine.

### Brief Description of Drawings

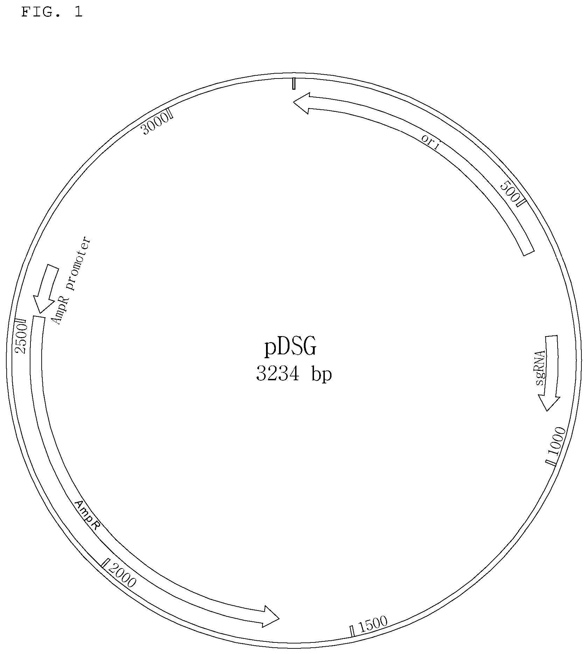

FIG. 1 shows the structure of plasmid pDSG according to one embodiment of the present invention.

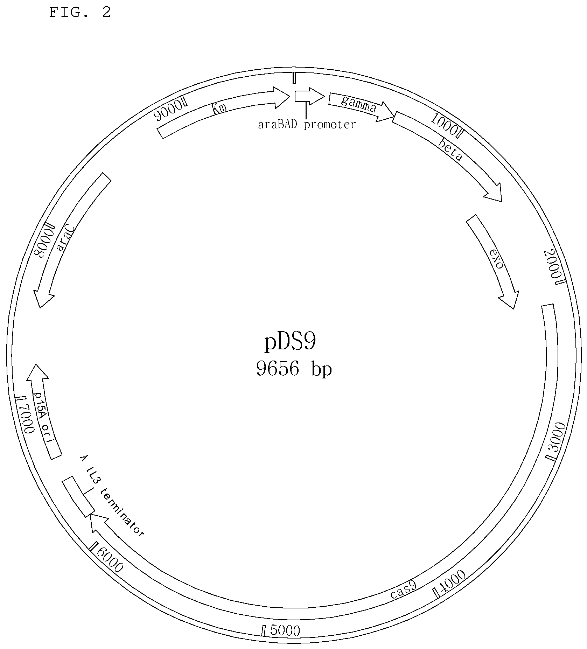

FIG. 2 shows the structure of plasmid pDS9 according to one embodiment of the present invention.

### Mode for Invention

Hereinafter, the present invention will be described in more detail. However, this description is merely presented by way of example to facilitate the understanding of the present invention, and the scope of the present invention is not limited by this exemplary description.

### Example 1. Construction of Strains Expressing Phenylalanine:H+ Symporter PheP Variant

In order to evaluate the effect of a variant (SEQ ID NO: 4) resulting from substitution of lysine (Lys) for glutamic acid (Glu) at position 272 in the amino acid sequence of phenylalanine:H+ symporter PheP (SEQ ID NO: 2) on the production of L-aromatic amino acids, a vector expressing the phenylalanine:H+ symporter PheP variant and strains into which the vector has been introduced were constructed. For insertion of the gene encoding the phenylalanine:H+ symporter PheP variant into the strains, plasmids pDSG and pDS9 were used, and the strains were constructed as follows.

The plasmid pDSG (SEQ ID NO: 23) has an origin of replication that works only in *Escherichia coli,* contains an ampicillin resistance gene, and has a mechanism for expressing guide RNA (gRNA). The plasmid pDS9 (SEQ ID NO: 24) has an origin of replication that works only in *Escherichia coli,* contains a kanamycin resistance gene and a system for expression of λ red genes (*exo, bet* and *gam*), and has a mechanism for expressing CAS9 derived from *Streptococcus pyogenes.*

### 1-1. Construction of Vector pDSG-pheP (Glu272Lys) for Transformation

Using *Escherichia coli* MG1655 (KCTC14419BP) gDNA as a template, PCR was performed using a primer pair of primer 7 and primer 9 and a primer pair of primer 8 and primer 10 to obtain an upstream fragment of pheP for the amino acid mutation at position 272 in the amino acid sequence of phenylalanine:H+ symporter PheP. In addition, using *E. coli* MG1655 (KCTC14419BP) gDNA as a template, PCR was performed using a primer pair of primer 11 and primer 13 and a primer pair of primer 12 and primer 14 to obtain a downstream fragment of pheP for the amino acid mutation at position 272 in the amino acid sequence of phenylalanine:H+ symporter PheP. Each of the upstream and downstream fragments contained a codon sequence that translates Glu at position 272 in the amino acid sequence of phenylalanine:H+ symporter PheP into Lys. In the PCR, Takara PrimeSTAR Max DNA polymerase was used as polymerase, and PCR was performed for 30 cycles under the following conditions: denaturation at 95°C for 10 sec, annealing at 57°C for 15 sec, and polymerization at 72°C for 10 sec.

Using the plasmid pDSG as a template, four pDSG gene fragments were obtained by performing PCR using a primer pair of primer 3 and primer 5, a primer pair of primer 4 and primer 6, a primer pair of primer 15 and primer 1, and a primer pair of primer 16 and primer 2, respectively. Each of the gene fragments contained a gRNA sequence targeting Glu of pheP. The gRNA was selected as a 20 mer upstream of NGG of the sequence to be mutated. In the PCR, Takara PrimeSTAR Max DNA polymerase was used as polymerase, and PCR was performed for 30 cycles under the following conditions: denaturation at 95°C for 10 sec, annealing at 57°C for 15 sec, and polymerization at 72°C for 15 sec.

Next, the obtained upstream and downstream fragments of pheP, and the obtained four fragments of pDSG were cloned using a self-assembly cloning method (BioTechniques 51:55-56 (July 2011)), thereby obtaining a recombinant plasmid which was named pDSG-pheP (Glu272Lys).

### 1-2. Construction of L-Tryptophan- or L-Phenylalanine-Producing Strain into Which Phenylalanine:H+ Symporter PheP Variant pheP (Glu272Lys) Has Been Introduced

To construct an L-tryptophan-producing strain and an L-phenylalanine-producing strain, *Escherichia coli* KCCM13013P and KCCM10016 were used as parent strains, respectively.

Each of the parent strains was primarily transformed with the plasmid pDS9 and cultured on LB-Km (containing 25 g/L of Luria Bertani broth and 50 mg/L of kanamycin) solid medium, and then colonies having kanamycin resistance were selected. The selected colonies were secondarily transformed with the pDSG-pheP (Glu272Lys) plasmid and cultured on LB-Amp & Km (containing 25 g/L of Luria Bertani broth, 100 mg/L of ampicillin and 50 mg/L of kanamycin) solid medium, and then ampicillin- and kanamycin-resistant colonies were selected and subjected to colony PCR using a primer pair of primer 17 and primer 18 to obtain a fragment. Here, Takara PrimeSTAR Max DNA polymerase was used as polymerase, and PCR was performed for 30 cycles under the following conditions: denaturation at 95°C for 10 sec, annealing at 57°C for 10 sec, and polymerization at 72°C for 15 sec. The fragment obtained using the primer pair of primer 17 and primer 18 was sequenced.

The selected secondary transformants were passaged 7 times in LB liquid medium, and colonies were selected on LB solid medium. Each colony was picked and cultured on each of LB, LB-Amp and LB-Km solid media. Colonies that did not grow on the LB-Amp and LB-Km solid media while growing on the LB solid medium were selected. The strains thus selected were named KCCM13013P_pheP (Glu272Lys) and KCCM10016_pheP (Glu272Lys), respectively.

The primer sequences used in Example 1 are shown in Table 1 below.

**[Table 1]**

| Primer name | SEQ ID NO. | Primer sequence (5' → 3') |
|---|---|---|
| Primer 1 | SEQ ID NO: 5 | caattttattatagtaattgactattatac |
| Primer 2 | SEQ ID NO: 6 | TGAAATCCAAcaattttattatagtaattgactattata c |
| Primer 3 | SEQ ID NO: 7 | TTGGATTTCACTTTCACCCAgttttagagctagaaatag c |
| Primer 4 | SEQ ID NO: 8 | CTTTCACCCAgttttagagctagaaatagc |
| Primer 5 | SEQ ID NO: 9 | gagcctgtcggcctacctgct |
| Primer 6 | SEQ ID NO: 10 | cggccggcatgagcctgtcg |
| Primer 7 | SEQ ID NO: 11 | atgccggccgCGCTTATATGGCGAAACCGA |
| Primer 8 | SEQ ID NO: 12 | CGCTTATATGGCGAAACCGAGTTCT |
| Primer 9 | SEQ ID NO: 13 | TAGAGCGCCAGTAAAACCACCAGTG |
| Primer 10 | SEQ ID NO: 14 | CACCCACGGATAGAGCGCCAGTAAA |
| Primer 11 | SEQ ID NO: 15 | TCCGTGGGTGGAAGTGAAATCCAACAGTAG |
| Primer 12 | SEQ ID NO: 16 | GAAGTGAAATCCAACAGTAGCCCGT |
| Primer 13 | SEQ ID NO: 17 | GATCATAATCCAGTTCAACAGCAGC |
| Primer 14 | SEQ ID NO: 18 | GCGCCAGACAGATCATAATCCAGTT |
| Primer 15 | SEQ ID NO: 19 | TGTCTGGCGCgagctcctgaaaatctcgataac |
| Primer 16 | SEQ ID NO: 20 | gagctcctgaaaatctcgataac |
| Primer 17 | SEQ ID NO: 21 | ATATGCAGTACTGGTTCCCG |
| Primer 18 | SEQ ID NO: 22 | AACTGTGTTTCACGCCCCTG |

### Experimental Example 1. Evaluation of L-Aromatic Amino Acid Productivity of Strain into Which Phenylalanine:H+ Symporter PheP Variant Has Been Introduced

The L-tryptophan or L-phenylalanine productivities of the parent strains (KCCM13013P and KCCM10016) and the strains (KCCM13013P_pheP (Glu272Lys) and KCCM10016_pheP (Glu272Lys)) into which the phenylalanine:H+ symporter PheP variant has been introduced were compared.

1 vol% of each strain (parent strain or mutant strain) was inoculated into a flask containing 10 mL of the medium for tryptophan production or medium for phenylalanine production shown in Table 2 below and was cultured with shaking at 37°C and 200 rpm for 72 hours. After completion of the culturing, the concentration of L-tryptophan or L-phenylalanine in the medium was measured using HPLC (Agilent), and the results are shown in Tables 3 and 4 below, respectively.

**[Table 2]**

| Medium for tryptophan production | | Medium for phenylalanine production | |
|---|---|---|---|
| Component | Content | Component | Content |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0 g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆Mo₇O₂₄ | 0.12 ppm | Yeast extract | 1.0 g/L |
| H₃BO₃ | 0.01 ppm | Glutamic acid | 0.5 g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-Tyrosine | 200.00 ppm | L-Tyrosine | 200.00 ppm |
| L-phenylalanine | 300.00 ppm | CaCO₃ | 3% |
| CaCO₃ | 3% | - | - |
| pH 7.0 with NaOH (33%) | | pH 7.0 with NaOH (33%) | |

**[Table 3]**

| Strain | L-tryptophan concentration (g/L) | L-tryptophan concentration increase rate (%) |
|---|---|---|
| KCCM13013P | 4.00 | - |
| KCCM13013P_pheP (Glu272Lys) | 4.45 | 11.3 |

**[Table 4]**

| Strain | L-phenylalanine concentration (g/L) | L-phenylalanine concentration increase rate (%) |
|---|---|---|
| KCCM10016 | 3.14 | - |
| KCCM10016_pheP (Glu272Lys) | 3.4 | 8.3 |

As shown in Tables 3 and 4 above, it was confirmed that the mutant strains KCCM13013P_pheP (Glu272Lys) and KCCM10016_pheP (Glu272Lys) into which the phenylalanine:H+ symporter PheP variant has been introduced showed increases in L-tryptophan and L-phenylalanine production of about 11.3% and 8.3%, respectively, compared to the parent strains, due to the substitution of lysine for glutamic acid at position 272 in the amino acid sequence of phenylalanine:H+ symporter PheP.

So far, the present invention has been described with reference to the embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all differences within a range equivalent to the scope of the appended claims should be construed as being included in the present invention.

### [Accession Numbers]

Depository Authority: Korean Collection for Type Cultures (KCTC), Biological Resource Center, Korea Research Institute of Bioscience and Biotechnology
Accession Number: KCTC14419BP
Deposit Date: December 28, 2020
Depository Authority: Korean Culture Center of Microorganisms (KCCM)
Accession Number: KCCM13013P
Deposit Date: June 22, 2021
Depository Authority: Korean Culture Center of Microorganisms (KCCM)
Accession Number: KCCM10016
Deposit Date: October 24, 1992

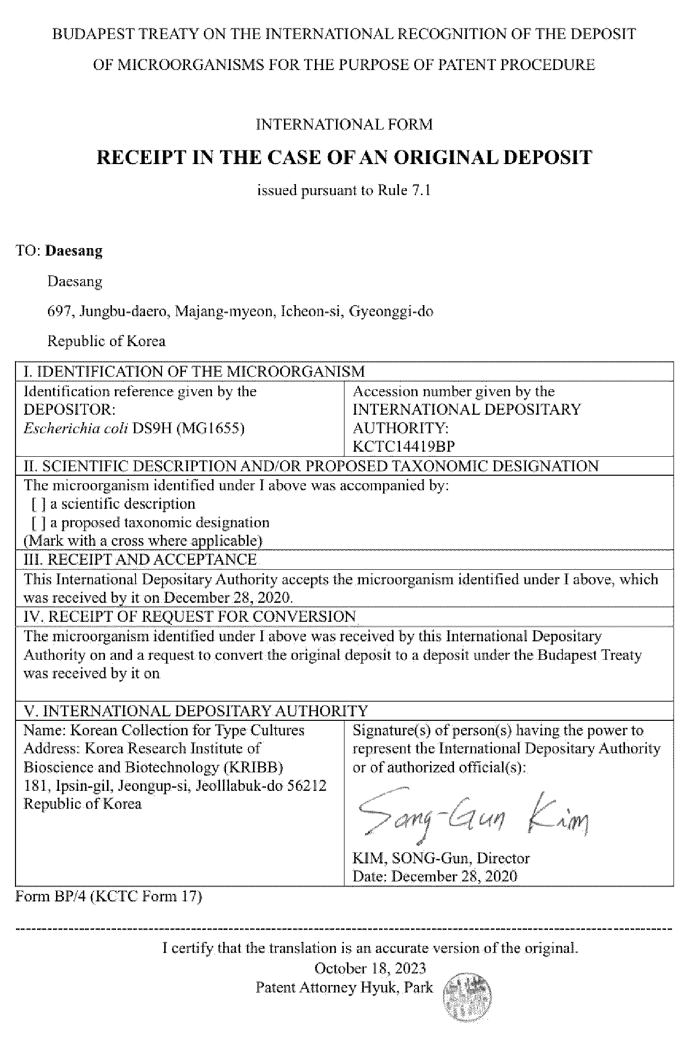
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM
RECEIPT IN THE CASE OF AN ORIGINAL DEPOSIT
issued pursuant to Rule 7.1

TO: Daesang
Daesang
697, Jungbu-daero, Majang-myeon, Icheon-si, Gyeonggi-do
Republic of Korea

I. IDENTIFICATION OF THE MICROORGANISM
Identification reference given by the
DEPOSITOR:
Escherichia coli DS9H (MG1655) | Accession number given by the
INTERNATIONAL DEPOSITARY
AUTHORITY:
KCTC14419BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which
was received by it on December 28, 2020.
IV. RECEIPT OF REQUEST FOR CONVERSION
The microorganism identified under I above was received by this International Depositary
Authority on and a request to convert the original deposit to a deposit under the Budapest Treaty
was received by it on
V. INTERNATIONAL DEPOSITARY AUTHORITY
Name: Korean Collection for Type Cultures
Address: Korea Research Institute of
Bioscience and Biotechnology (KRIBB)
181, Ipsin-gil, Jeongup-si, Jeolllabuk-do 56212
Republic of Korea | Signature(s) of person(s) having the power to
represent the International Depositary Authority
or of authorized official(s):
KIM, SONG-Gun, Director
Date: December 28, 2020
Form BP/4 (KCTC Form 17)

I certify that the translation is an accurate version of the original.
October 18, 2023
Patent Attorney Hyuk, Park

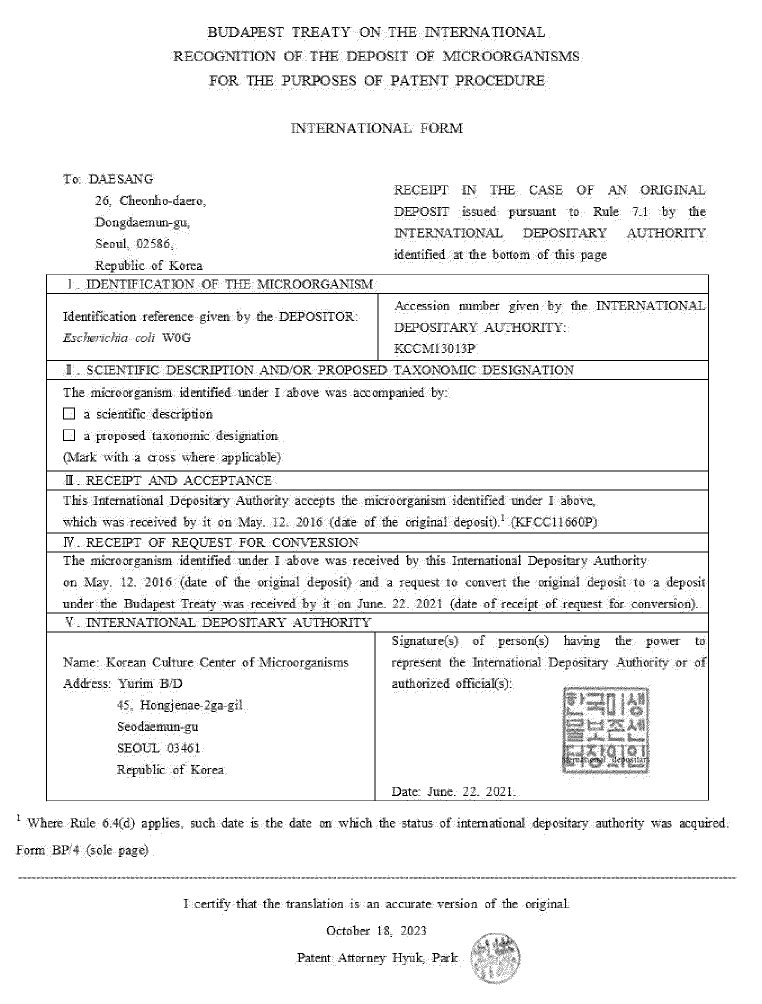
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
26, Cheonho-daero,
Dongdaemun-gu,
Seoul, 02586,
Republic of Korea

RECEIPT IN THE CASE OF AN ORIGINAL
DEPOSIT issued pursuant to Rule 7.1 by the
INTERNATIONAL DEPOSITARY AUTHORITY
identified at the bottom of this page

Ⅰ. IDENTIFICATION OF THE MICROORGANISM
Identification reference given by the DEPOSITOR:
Escherichia coli W0G | Accession number given by the INTERNATIONAL
DEPOSITARY AUTHORITY:
KCCM13013P
Ⅱ. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
Ⅲ. RECEIPT AND ACCEPTANCE
This International Depositary Authority accepts the microorganism identified under I above,
which was received by it on May. 12. 2016 (date of the original deposit).1 (KFCC11660P)
Ⅳ. RECEIPT OF REQUEST FOR CONVERSION
The microorganism identified under I above was received by this International Depositary Authority
on May. 12. 2016 (date of the original deposit) and a request to convert the original deposit to a deposit
under the Budapest Treaty was received by it on June. 22. 2021 (date of receipt of request for conversion).
Ⅴ. INTERNATIONAL DEPOSITARY AUTHORITY
Name: Korean Culture Center of Microorganisms
Address: Yurim B/D
45, Hongjenae-2ga-gil
Seodaemun-gu
SEOUL 03461
Republic of Korea | Signature(s) of person(s) having the power to
represent the International Depositary Authority or of
authorized official(s):
Date: June. 22. 2021.

1 Where Rule 6.4(d) applies, such date is the date on which the status of international depositary authority was acquired.
Form BP/4 (sole page)

I certify that the translation is an accurate version of the original.
October 18, 2023
Patent Attorney Hyuk, Park

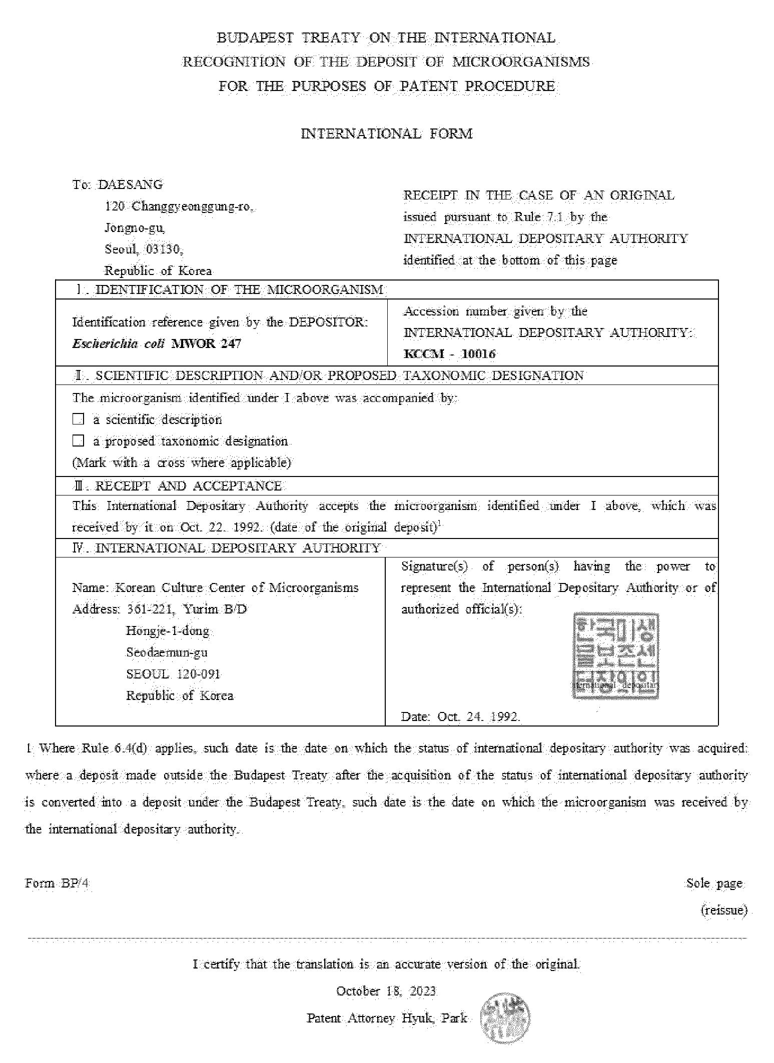
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To: DAESANG
120 Changgyeonggung-ro,
Jongno-gu,
Seoul, 03130,
Republic of Korea

RECEIPT IN THE CASE OF AN ORIGINAL
issued pursuant to Rule 7.1 by the
INTERNATIONAL DEPOSITARY AUTHORITY
identified at the bottom of this page

Ⅰ. IDENTIFICATION OF THE MICROORGANISM
Identification reference given by the DEPOSITOR:
Escherichia coli MWOR 247 | Accession number given by the
INTERNATIONAL DEPOSITARY AUTHORITY:
KCCM - 10016
Ⅱ. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
Ⅲ. RECEIPT AND ACCEPTANCE
This International Depositary Authority accepts the microorganism identified under I above, which was received by it on Oct. 22. 1992. (date of the original deposit)¹
Ⅳ. INTERNATIONAL DEPOSITARY AUTHORITY
Name: Korean Culture Center of Microorganisms
Address: 361-221, Yurim B/D
Hongje-1-dong
Seodaemun-gu
SEOUL 120-091
Republic of Korea | Signature(s) of person(s) having the power to represent the International Depositary Authority or of authorized official(s):
Date: Oct. 24. 1992.

1 Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authority.

Form BP/4
Sole page
(reissue)

I certify that the translation is an accurate version of the original.
October 18, 2023
Patent Attorney Hyuk, Park

## Claims

1. A phenylalanine:H+ symporter PheP variant consisting of the amino acid sequence of SEQ ID NO: 4 in which glutamic acid (Glu) at position 272 in the amino acid sequence of SEQ ID NO: 2 is substituted with lysine (Lys).

2. A polynucleotide encoding the variant of claim 1.

3. A transformant comprising the variant of claim 1 or the polynucleotide of claim 2.

4. The transformant of claim 3, which is an *Escherichia* sp. strain or a *Corynebacterium* sp. strain.

5. The transformant of claim 3, which has ability to produce an L-aromatic amino acid.

6. A method for producing an L-aromatic amino acid, comprising steps of:
culturing the transformant of claim 3 in a medium; and
recovering an L-aromatic amino acid from the transformant or the medium in which the transformant has been cultured.

7. The method of claim 6, wherein the L-aromatic amino acid is at least one selected from the group consisting of L-tryptophan, L-phenylalanine, and L-tyrosine.
